# EUROPEAN PATENT APPLICATION

(11) **EP 4 596 800 A1**
(43) Date of publication of application: **06.08.2025**
(21) Application number: 23871456.2
(22) Date of filing: 02.08.2023
(51) Int. Cl.: E02F 9/20, E02F 9/26

(54) **WORK MACHINE MANAGEMENT DEVICE AND MANAGEMENT SYSTEM**

(30) Priority: 28.09.2022 JP 2022155570
(71) Applicant: Hitachi Construction Machinery Co., Ltd., Tokyo 110-0015 (JP)
(72) Inventor: KURASAKO, Akira, Tsuchiura-shi, Ibaraki 300-0013 (JP); AKITA, Hideki, Tsuchiura-shi, Ibaraki 300-0013 (JP)
(74) Representative: MERH-IP Matias Erny Reichl Hoffmann Patentanwälte PartG mbB
(86) International application number: PCT/JP2023/028321
(87) International publication number: WO 2024/070214

(57) **Abstract**

The present invention provides a management device and management system for work machine that can improve the accuracy of determining oil abnormalities. The management server manages the oil used in the drive device of a hydraulic excavator. Additionally, the management server has an oil change detection unit that obtains measurement values of the properties of the target oil from an oil sensor mounted on the hydraulic excavator and detects the exchange of the target oil based on changes in the properties of the target oil. Furthermore, the management server has an oil type candidate selection unit that selects multiple oil type candidates for the exchanged target oil based on the properties measured by the oil sensor after detecting the exchange of the target oil. Additionally, the management server has an oil type acquisition unit that outputs a command to select the type of the target oil from the multiple oil type candidates and acquires the type of the target oil by accepting the selection input of the type of the target oil. Then, the management server has an oil abnormality judgment unit that determines abnormalities in the target oil by comparing the properties measured by the oil sensor with a threshold corresponding to the type of the target oil.

## Description

### Technical Field

The present invention relates to a management device and management system for work machine.

### Background Art

The drive device mounted on a work machine includes, for example, an engine, a circulation system for circulating engine oil used for lubrication and cooling of the engine, a hydraulic pump driven by the engine, and a hydraulic actuator driven by hydraulic oil discharged from the hydraulic pump.

Patent Document 1 discloses a server computer that targets the oil used in the drive device of a work machine (specifically, the aforementioned engine oil and hydraulic oil) and collects the properties of the oil measured by the oil sensor of the work machine. This server computer determines oil abnormalities by comparing the properties of the oil measured by the oil sensor with a predetermined threshold.

### Prior Art Document

### Patent Documents

Patent Document 1: Japanese Patent No. 6234359

### Summary of the Invention

### Problem to be Solved by the Invention

Although it is preferable to use genuine oil (in other words, oil recommended by the manufacturer of the work machine) in the drive device of a work machine, non-genuine oil (in other words, oil not recommended by the manufacturer of the work machine) may be used. One method for determining the type of oil used in the drive device of a work machine is to use the measurement results of an oil sensor. Specifically, the computer detects oil changes based on changes in the properties of the oil measured by the oil sensor. Then, the type of oil is determined based on the properties measured by the oil sensor after the oil change.

However, in the aforementioned method, even immediately after an oil change, variations in properties may occur due to individual differences in the oil or the influence of residual oil, which may reduce the accuracy of determining the type of oil. Therefore, the threshold for determining oil abnormalities may not be optimized, potentially reducing the accuracy of determining oil abnormalities.

The purpose of the present invention is to provide a management device and management system for work machine that can improve the accuracy of determining oil abnormalities.

### Means for Solving the Problem

To achieve the above objective, a management device, which is a representative embodiment of the present invention for managing the oil used in the drive device of a work machine, obtains measurement values of the properties of the target oil from an oil sensor mounted on the work machine and has an oil change detection unit that detects the exchange of the target oil based on changes in the properties of the target oil. Furthermore, the management device has an oil type candidate selection unit that selects multiple oil type candidates for the exchanged target oil based on the properties measured by the oil sensor after detecting the exchange of the target oil. Additionally, the management device has an oil type acquisition unit that outputs a command to select the type of the target oil from the multiple oil type candidates and acquires the type of the target oil by accepting the selection input of the type of the target oil. Then, the management device has an oil abnormality judgment unit that determines abnormalities in the target oil by comparing the properties measured by the oil sensor with a threshold corresponding to the type of the target oil.

### Advantages of the Invention

According to the present invention, the accuracy of determining oil abnormalities can be improved.

### Brief Description of the Drawings

[FIG. 1] is a block diagram illustrating the configuration of a management system in an embodiment of the present invention.
[FIG. 2] is a side view illustrating the construction of a hydraulic excavator in an embodiment of the present invention.
[FIG. 3] is a schematic diagram illustrating the configuration of a drive device for a hydraulic excavator in an embodiment of the present invention.
[FIG. 4] is a flowchart showing the process related to the acquisition of oil types by the management controller of the management server according to an embodiment of the present invention.
[FIG. 5] is a diagram for explaining the detection of oil exchange by the management controller of the management server according to an embodiment of the present invention.
[FIG. 6] is a diagram showing the screen of a monitor of a hydraulic excavator or the screen of a terminal for workers according to an embodiment of the present invention.
[FIG. 7] is a diagram for explaining the determination of oil abnormalities by the management controller of the management server according to an embodiment of the present invention.

### Mode for Carrying Out the Invention

An embodiment of the present invention will be described with reference to the drawings. In this embodiment, a hydraulic excavator is taken as an example of a work machine.

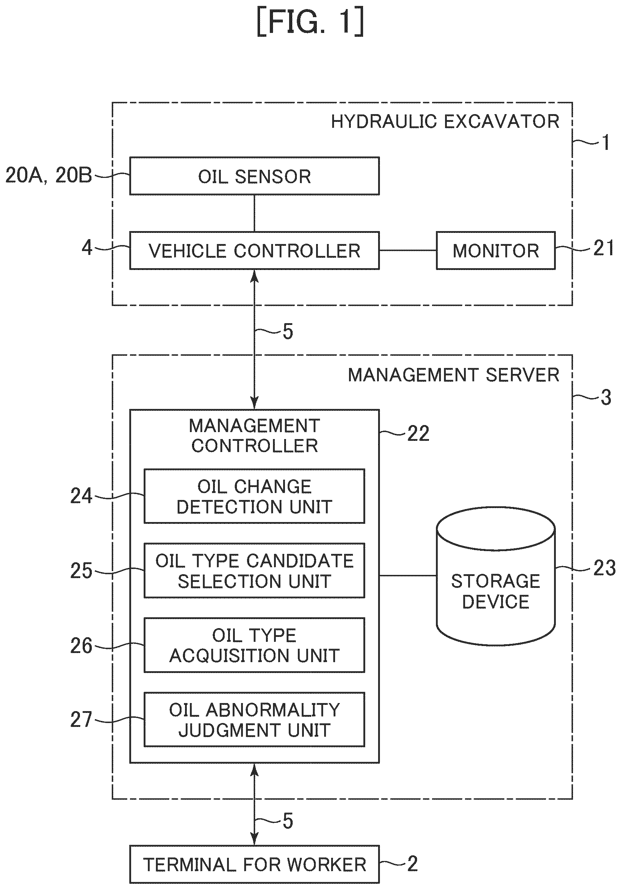

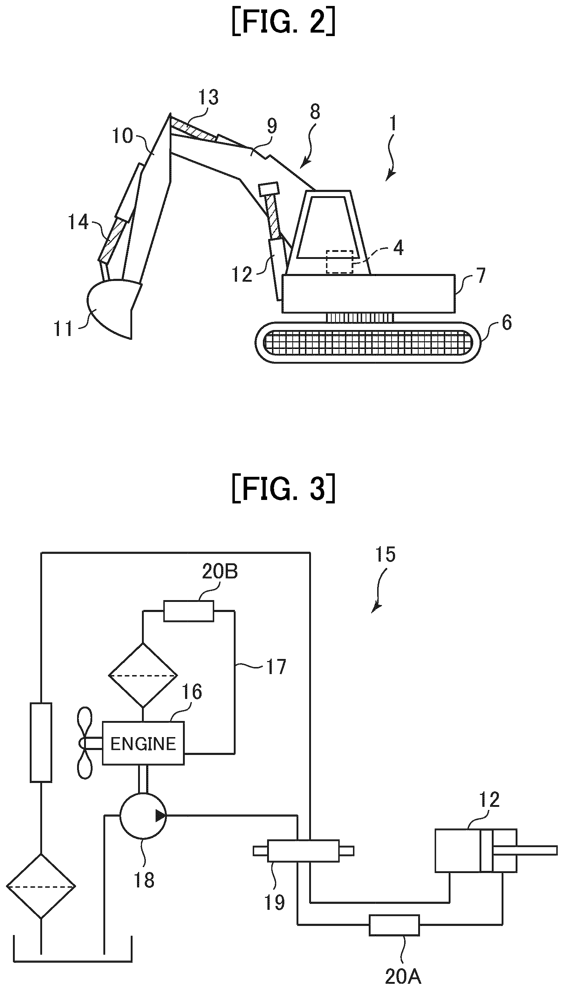

FIG. 1 is a block diagram showing the configuration of the management system in this embodiment. FIG. 2 is a side view showing the structure of the hydraulic excavator in this embodiment. FIG. 3 is a schematic diagram showing the configuration of the drive device of the hydraulic excavator in this embodiment. In FIG. 3, although only the configuration related to the drive of the boom cylinder is shown for convenience among the configurations related to the drive of multiple hydraulic actuators (specifically, multiple control valves, etc.), the configuration related to the drive of other hydraulic actuators is the same.

The management system of this embodiment includes a hydraulic excavator 1, a terminal 2 held by a worker who performs maintenance on the hydraulic excavator 1, and a management server 3 held by the manufacturer of the hydraulic excavator 1. The vehicle controller 4 mounted on the hydraulic excavator 1, the terminal 2 for workers, and the management server 3 can communicate with each other via a communication network 5.

The hydraulic excavator 1 includes a drivable lower travel body 6, an upper swing body 7 pivotably provided on the upper side of the lower travel body 6, and a working device 8 connected to the upper swing body 7. The lower travel body 6 travels by the rotation of a travel motor (not shown), and the upper swing body 7 swings by the rotation of a swing motor (not shown).

The working device 8 includes a boom 9 pivotably connected to the upper swing body 7, an arm 10 pivotably connected to the distal end of the boom 9, and a bucket 11 pivotably connected to the distal end of the arm 10. The boom 9 rotates by the expansion and contraction of the boom cylinder 12, the arm 10 rotates by the expansion and contraction of the arm cylinder 13, and the bucket 11 rotates by the expansion and contraction of the bucket cylinder 14.

The drive device 15 of the hydraulic excavator 1 includes an engine 16, a circulation system 17 for circulating engine oil used for lubrication and cooling of the engine 16, a hydraulic pump 18 driven by the engine 16, multiple hydraulic actuators driven by hydraulic oil discharged from the hydraulic pump 18 (specifically, the aforementioned travel motor, swing motor, boom cylinder 12, arm cylinder 13, and bucket cylinder 14), multiple control valves 19 for controlling the flow of pressure oil from the hydraulic pump 18 to the multiple hydraulic actuators, and multiple operating devices (not shown) for operating the multiple control valves 19.

In this embodiment, the oil sensor 20A is provided, for example, in the oil passage between the boom cylinder 12 and the control valve 19, and measures the properties of the hydraulic oil (specifically, for example, viscosity, density, dielectric constant, chromaticity, and particle concentration, etc.). The oil sensor 20B is provided in the circulation system 17 and measures the properties of the engine oil.

Although not shown, the vehicle controller 4 of the hydraulic excavator 1 includes a processor that executes processing according to a program, and a memory that stores programs and data. The vehicle controller 4 temporarily stores the properties of the hydraulic oil measured by the oil sensor 20A and the properties of the engine oil measured by the oil sensor 20B in memory, and transmits them to the management server 3 at a predetermined cycle.

The monitor 21 of the hydraulic excavator 1 is arranged in the cab of the hydraulic excavator 1 and is connected to the vehicle controller 4 via wiring. Although not shown, the monitor 21 includes a processor that executes processing according to a program, a memory that stores programs and data, and an interface capable of input and display.

Although not shown, the terminal 2 for workers includes a processor that executes processing according to a program, a memory that stores programs and data, and an interface capable of input and display.

The management server 3 of this embodiment targets the oil used in the drive device 15 of the hydraulic excavator 1 (specifically, the aforementioned hydraulic oil and engine oil) and has the function of collecting the properties of the oil measured by the oil sensors 20A and 20B of the hydraulic excavator 1, corresponding to the management device described in the claims. In the following, the oil to be measured by the oil sensor 20A or 20B (i.e., the oil to be managed) may be referred to as the target oil, and in this embodiment, the hydraulic oil or engine oil is the target oil.

The management server 3 includes a management controller 22 and a storage device 23. Although not shown, the management controller 22 includes a processor that executes processing according to a program, and a memory that stores programs and data. The memory of the management controller 22 temporarily stores time-series data of the properties of the target oil obtained from the vehicle controller 4. The processor of the management controller 22 processes the data stored in the memory and stores the data created by the processing in the storage device 23.

The storage device 23 is, for example, a hard disk that constructs a database, and stores the data of the hydraulic excavator 1 obtained from the vehicle controller 4, the data obtained from the terminal 2 for workers, the data created by the management controller 22, and the data of multiple types of oil obtained in advance (specifically, the initial properties for each oil type and the threshold of properties set in advance for abnormality determination for each oil type).

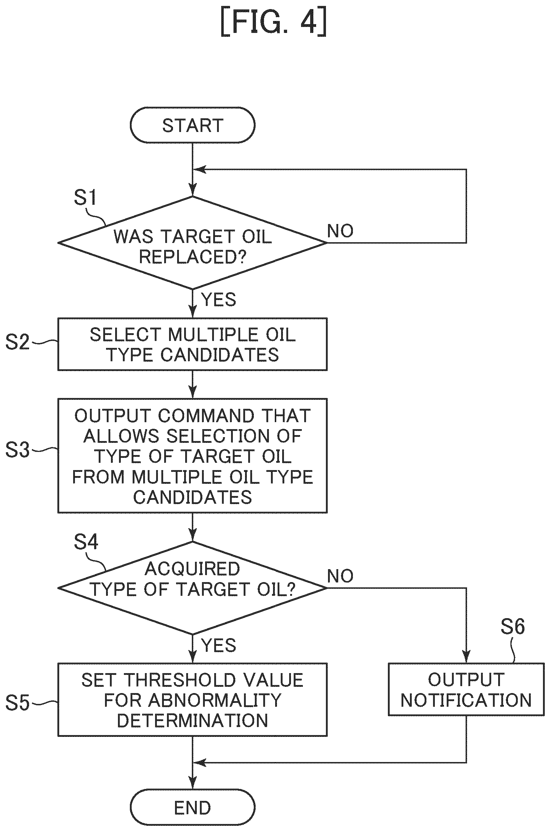

The management controller 22 has, as a functional configuration, an oil change detection unit 24, an oil type candidate selection unit 25, an oil type acquisition unit 26, and an oil abnormality determination unit 27. The processing content of this management controller 22 will be described. FIG. 4 is a flowchart showing the process related to the acquisition of the type of target oil in the processing of the management controller 22 of this embodiment.

The oil change detection unit 24 of the management controller 22 detects the replacement of the target oil using the time-series data of the target oil obtained from the vehicle body controller 4 (Step S1 in FIG. 4).

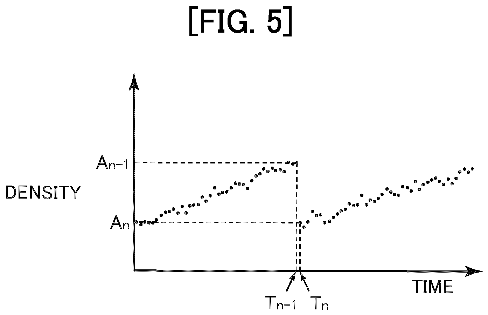

Specifically, as shown in FIG. 5, if the change amount (An-An-1) of the properties of the target oil (although density is shown as an example in FIG. 5, other properties may also be used) exceeds a predetermined value (specifically, a value set in advance considering the deterioration of the target oil and the measurement error of the oil sensor), it is determined that the period from time Tn-1 to time Tn is the oil change period (i.e., the target oil was replaced during this period). Then, the replacement history of the target oil for the hydraulic excavator 1 (e.g., the oil change date) is created and stored in the storage device 23.

The oil type candidate selection unit 25 of the management controller 22 extracts the properties of the target oil immediately after replacement (An in FIG. 5) from the time-series data of the properties of the target oil obtained from the vehicle body controller 4. Then, based on the properties of the target oil immediately after replacement and the data of multiple types of oil stored in the storage device 23, multiple oil type candidates are selected (Step S2 in FIG. 4). Specifically, multiple first oil type candidates having properties within a preset first range (e.g., 80% to 120%) relative to the properties of the target oil immediately after replacement are selected. Additionally, at least one second oil type candidate having properties outside the first range and within a preset second range (e.g., 70% to 130%) that is larger than the first range is selected relative to the properties of the target oil immediately after replacement.

The oil type acquisition unit 26 of the management controller 22 outputs a command that allows the selection of the type of target oil from the aforementioned multiple oil type candidates to the monitor 21 via the vehicle body controller 4 of the hydraulic excavator 1, or to the worker's terminal 2 (Step S3 in FIG. 4).

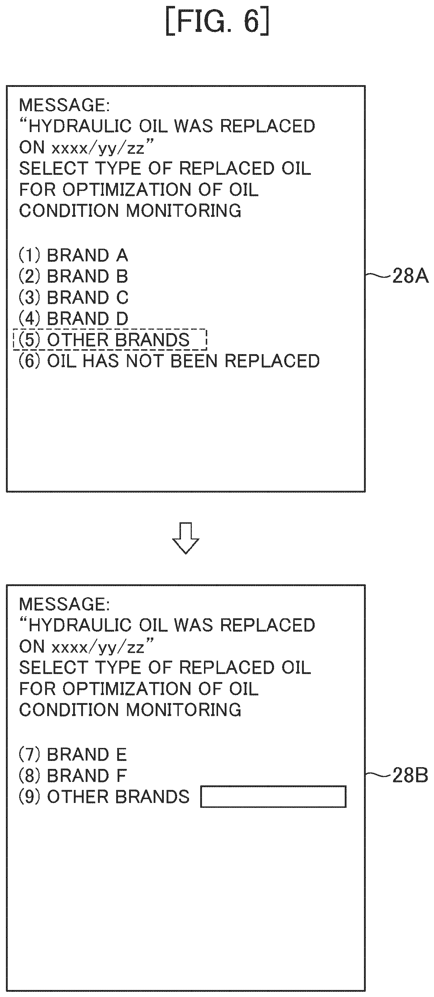

The monitor 21 or terminal 2 displays multiple oil type candidates in response to the aforementioned command. Specifically, as shown in FIG. 6, on the first stage screen 28A, multiple first oil type candidates "Brand A," "Brand B," "Brand C," and "Brand D" are displayed as selectable options along with "Other Brands" and "Oil has not been replaced." "Brand A," "Brand B," "Brand C," and "Brand D" may be displayed in the order of having properties close to those of the target oil immediately after replacement, for example. If "Other Brands" on screen 28A is selected by the operator of the hydraulic excavator 1 operating the monitor 21 or by a worker operating the terminal 2, the display transitions to the second stage screen 28B, where the second oil type candidates "Brand E" and "Brand F" are displayed as selectable options along with "Other Brands." "Brand E" and "Brand F" may be displayed in the order of having properties close to those of the target oil immediately after replacement, for example. If "Other Brands" on screen 28B is selected, it allows for the input of a new brand.

If any of "Brand A," "Brand B," "Brand C," "Brand D," "Brand E," or "Brand F" is selected as the type of target oil, the monitor 21 or terminal 2 outputs the type of target oil to the management controller 22 of the management server 3. As a result, the oil type acquisition unit 26 of the management controller 22 acquires the type of target oil and stores it in the storage device 23 while associating it with the replacement history of the target oil.

In this case, the oil abnormality judgment unit 27 of the management controller 22 reads and sets the threshold values of the properties corresponding to the type of target oil from the storage device 23 (Step S5 in FIG. 4). Then, by comparing the time-series data of the properties of the target oil obtained from the vehicle body controller 4 of the hydraulic excavator 1 with the threshold values, the abnormality of the target oil is determined.

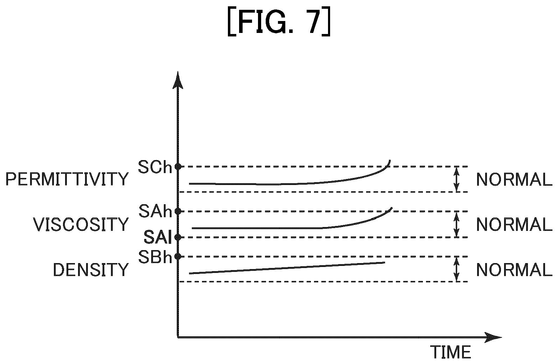

In detail, if the target oil is Brand A, the oil abnormality judgment unit 27 of the management controller 22 reads and sets the corresponding upper viscosity threshold SAh and lower viscosity threshold SAl, upper density threshold SBh, and upper dielectric constant threshold SCh from the storage device 23. Then, as shown in FIG. 7, if the viscosity of the target oil exceeds the upper threshold SAh, it is determined to be abnormal. Alternatively, if the viscosity of the target oil falls below the lower threshold SAl, it is determined to be abnormal. Also, if the density of the target oil exceeds the upper threshold SBh, it is determined to be abnormal. Additionally, as shown in FIG. 7, if the dielectric constant of the target oil exceeds the upper threshold SCh, it is determined to be abnormal.

If a new brand is entered as the type of target oil, the monitor 21 or terminal 2 outputs the type of target oil to the management server 3. As a result, the oil type acquisition unit 26 of the management controller 22 acquires the type of target oil and stores it in the storage device 23 while associating it with the replacement history of the target oil.

In this case, the oil abnormality judgment unit 27 of the management controller 22 selects an alternative oil type having the properties closest to those of the target oil immediately after replacement and reads and sets the threshold values of the properties corresponding to the alternative oil type from the storage device 23 (Step S5 in FIG. 4). Then, by comparing the time-series data of the properties of the target oil obtained from the vehicle body controller 4 of the hydraulic excavator 1 with the threshold values, the abnormality of the target oil is determined.

If "Oil has not been replaced" is selected, the monitor 21 or terminal 2 outputs that information to the management server 3. As a result, the management controller 22 of the management server 3 does not acquire the type of target oil and corrects the replacement history of the target oil stored in the storage device 23. Additionally, the management controller 22 of the management server 3 determines an oil change detection abnormality based on the aforementioned information and outputs the notification to the terminal 2 (Step S6 in FIG. 4). This allows the worker to investigate the cause of the oil change detection abnormality.

As described above, in this embodiment, the oil type candidate selection unit 25 of the management controller 22 selects multiple oil type candidates for the replaced target oil based on the properties measured by the oil sensor after detecting the replacement of the target oil. The oil type acquisition unit 26 of the management controller 22 outputs a command that allows the selection of the type of target oil from multiple oil type candidates and acquires the type of target oil by accepting the selection input of the type of target oil. The oil abnormality judgment unit 27 of the management controller 22 determines the abnormality of the target oil by comparing the properties measured by the oil sensor with the threshold values corresponding to the type of target oil. Therefore, the threshold values for determining oil abnormalities can be optimized, improving the accuracy of determining oil abnormalities.

It should be noted that, although not specifically described in the above embodiment, the hydraulic excavator 1 may be equipped with a temperature sensor (not shown) for measuring the temperature of the target oil. The vehicle body controller 4 of the hydraulic excavator 1 may temporarily store the temperature of the target oil measured by the temperature sensor in memory and transmit it to the management server 3 at a predetermined interval. The management controller 22 of the management server 3 may correct the properties of the target oil according to its temperature. That is, the properties of the target oil corresponding to its reference temperature may be calculated.

Additionally, although not specifically described in the above embodiment, the management controller 22 of the management server 3 may construct a function to correct the measurement values of the oil sensor based on the difference between the properties measured by the oil sensor and those stored in the storage device 23, and use this to correct the measurement values of the oil sensor.

Furthermore, in the above embodiment, the oil type candidate selection unit 25 of the management controller 22 was described as selecting at least one second oil type candidate having properties outside the first range and within a preset second range that is larger than the first range relative to the properties of the target oil immediately after replacement, but this is not limiting. The oil type candidate selection unit 25 of the management controller 22 may, for example, select at least one second oil type candidate that is different from the multiple first oil type candidates but has a usage history. In this case, the monitor 21 or terminal 2 may display the second oil type candidates, for example, in the order of proximity of usage period or frequency of use.

Additionally, in the above embodiment, the case where the management controller 22 of the management server 3 has the oil change detection unit 24, oil type candidate selection unit 25, and oil type acquisition unit 26 was described as an example, but this is not limiting. For example, the vehicle body controller 4 of the hydraulic excavator 1 may have the oil change detection unit 24, and the management controller 22 of the management server 3 may have the oil type candidate selection unit 25 and oil type acquisition unit 26. Alternatively, for example, the vehicle body controller 4 of the hydraulic excavator 1 may have the oil change detection unit 24, oil type candidate selection unit 25, and oil type acquisition unit 26.

It should be noted that, in the above, the case where the work machine is a hydraulic excavator was used as an example, but this is not limiting, and it may be, for example, a dump truck, wheel loader, bulldozer, forklift, or crane. Additionally, the oil used in the drive device of the work machine was described as being both hydraulic oil and engine oil as an example, but this is not limiting, and it may be only one of hydraulic oil and engine oil, or it may be another type of oil. That is, the oil to be managed (target oil) can be various type of oil. In that case, various information can be obtained from the work machine equipped with an oil sensor capable of measuring the properties of the target oil.

### Description of Reference Characters

1: Hydraulic excavator (work machine)
2: Terminal
3: Management server (management device)
15: Drive device
20A, 20B: Oil sensor
21: Monitor
24: Oil change detection unit
25: Oil type candidate selection unit
26: Oil type acquisition unit
27: Oil abnormality judgment unit

## Claims

1. A management device that manages the oil used in the drive device of the work machine, comprising:
an oil change detection unit that acquires measurement values of the properties of the target oil from the oil sensor mounted on the work machine and detects the replacement of the target oil based on changes in the properties of the target oil;
an oil type candidate selection unit that selects multiple oil type candidates for the replaced target oil based on the properties measured by the oil sensor after detecting the replacement of the target oil;
an oil type acquisition unit that outputs a command to select the type of the target oil from the multiple oil type candidates and acquires the type of the target oil by accepting the selection input of the type of the target oil; and
an oil abnormality judgment unit that determines the abnormality of the target oil by comparing the properties measured by the oil sensor with a threshold corresponding to the type of the target oil.

2. The management device for the work machine according to claim 1, wherein
the oil type candidate selection unit is configured to
select multiple first oil type candidates having properties within a preset first range with respect to the properties measured by the oil sensor after the replacement of the target oil, and
select at least one second oil type candidate having properties outside the first range and within a preset second range that is larger than the first range with respect to the properties measured by the oil sensor after the replacement of the target oil,
the oil type acquisition unit is configured to
output a command to, as a first stage, display the multiple first oil type candidates and allow the selection of the type of the target oil from the multiple first oil type candidates, and as a second stage, display the at least one second oil type candidate and allow the selection of the type of the target oil from the at least one second oil type candidate.

3. The management device for the work machine according to claim 1, wherein
the oil type candidate selection unit is configured to
select multiple first oil type candidates having properties within a preset range with respect to the properties measured by the oil sensor after the replacement of the target oil, and
select at least one second oil type candidate that is different from the multiple first oil type candidates but has a usage history,
the oil type acquisition unit is configured to
output a command to, as a first stage, display the multiple first oil type candidates and allow the selection of the type of the target oil from the multiple first oil type candidates, and as a second stage, display the at least one second oil type candidate and allow the selection of the type of the target oil from the at least one second oil type candidate.

4. The management device for the work machine according to claim 1, wherein
the oil abnormality judgment unit is configured to
select an alternative oil type having the closest properties with respect to the properties measured by the oil sensor after the replacement of the target oil when the type of the target oil acquired by the oil type acquisition unit is different from the multiple oil type candidates, and
determine the abnormality of the target oil by comparing the properties measured by the oil sensor with a threshold corresponding to the alternative oil type.

5. A management system comprising:
a work machine equipped with a drive device and an oil sensor that measures the properties of the oil used in the drive device; and
a management device that is capable of communicating with the work machine and manages the oil used in the drive device, wherein
the management device includes
an oil change detection unit that acquires measurement values of the properties of the target oil from the oil sensor mounted on the work machine and detects the replacement of the target oil based on changes in the properties of the target oil,
an oil type candidate selection unit that selects multiple oil type candidates for the replaced target oil based on the properties measured by the oil sensor after detecting the replacement of the target oil,
an oil type acquisition unit that outputs a command to select the type of the target oil from the multiple oil type candidates and acquires the type of the target oil by accepting the selection input of the type of the target oil, and
an oil abnormality judgment unit that determines the abnormality of the target oil by comparing the properties measured by the oil sensor with a threshold corresponding to the type of the target oil.

6. The management system for the work machine according to claim 5, wherein
the work machine is equipped with a monitor, and
the oil type acquisition unit of the management device outputs the command to the monitor of the work machine.

7. The management system for the work machine according to claim 5, further comprising
a terminal that is held by a worker performing maintenance on the work machine and is capable of communicating with the management device, wherein
the oil type acquisition unit of the management device outputs the command to the terminal.
